# EUROPEAN PATENT APPLICATION

(11) **EP 2 377 456 A1**
(43) Date of publication of application: **19.10.2011**
(21) Application number: 09833361.0
(22) Date of filing: 09.12.2009
(51) Int. Cl.: A61B 1/00

(54) **OPTICAL PROBE AND OPTICAL OBSERVATION DEVICE**

(30) Priority: 18.12.2008 JP 2008322842
(71) Applicant: FUJIFILM Corporation, Tokyo 106-8620 (JP)
(72) Inventor: TERAMURA, Yuichi, Ashigarakami-gun Kanagawa 258-8538 (JP)
(74) Representative: Klunker . Schmitt-Nilson . Hirsch
(86) International application number: PCT/JP2009/070584
(87) International publication number: WO 2010/071057

(57) **Abstract**

One aspect of this invention provides an optical probe (600) that includes: a tubular sheath (622) having a distal end that is closed; a light guiding means (623) provided inside the sheath, and arranged along the longitudinal axis of the sheath; and a light condensing means (628) that is provided on an emitting end side of the light guiding means, and that deflects an optical path of a light emitted from the emitting end to cause the light to converge on a measurement target. The optical probe (600) scans a convergent light emitted from the light condensing means (628) on a plane that is substantially orthogonal to an optical axis direction of the convergent light, and guides a light from the measurement target (S) to the light guiding means (623) via the light condensing means. The optical probe (600) also includes a cylindrical tube member (610) that has an outer circumferential wall that is integrally joined to an outer wall of the sheath (622) along the longitudinal axis direction.

## Description

### Technical Field

The present invention relates to an optical probe and an optical observation device, and more particularly to an optical probe and an optical observation device in which a sheath structure of the optical probe is a feature.

### Background Art

Conventionally, as an endoscope apparatus for observing the inside of a body cavity of a living organism, an electronic endoscope apparatus which emits illuminating light inside the body cavity of the living organism, picks up an image using reflected light, and displays the image on a monitor or the like is in widespread use, and is being utilized in various fields. Most endoscope apparatuses are equipped with a forceps opening, and it is possible to perform a biopsy or treatment of tissue inside the body cavity by means of a probe that is introduced into the body cavity through the forceps opening.

Meanwhile, the development of a tomographic image acquisition apparatus as an optical observation device which acquires a tomographic image of a living organism or the like without cutting a measurement target such as living tissue has been proceeding in recent years. For example, an optical tomographic imaging apparatus which utilizes an optical coherence tomography (OCT) measurement method that uses interference caused by low-coherence light is known (PTL 1).

The OCT measurement is a measurement method that utilizes a fact that interference light is detected when the optical path lengths of a measuring light and a reflected light match the optical path length of a reference light. That is, according to this method, low coherence light emitted from a light source is divided into measuring light and reference light, the measuring light is emitted to a measurement target, and reflected light from the measurement target is led to a multiplexing means. The reference light is led to the multiplexing means after the optical path length thereof is changed in order to change the depth of measurement in the measurement target. The reflected light and the reference light are multiplexed by the multiplexing means, and interference light produced as a result of the multiplexing is measured by heterodyne detection or the like.

In the above OCT apparatus, a tomographic image is acquired by changing the optical path length of the reference light, to thereby change the measuring position (measurement depth) within the measurement target. This technique is generally referred to as "TD-OCT (time domain OCT) measurement".

On the other hand, an optical tomographic imaging apparatus that utilizes SD-OCT (Spectral Domain OCT) measurement or SS-OCT (Swept Source OCT) measurement has been proposed as an apparatus for acquiring tomographic images at high speed without changing the optical path length of the reference light.

With respect to the above described tomographic images, a two-dimensional or three-dimensional optical tomographic image of a predetermined scanning area can be acquired by repeating measurement while shifting the irradiation position slightly each time.

This kind of OCT apparatus (optical tomographic imaging apparatus) enables detailed (a resolution of approximately 10 µm) observation of a measurement site. For example, invasion depth diagnosis of early cancers is enabled by inserting an OCT probe (optical probe) into the forceps opening of the endoscope apparatus, and guiding a signal light and a reflected light of the signal light to acquire an optical tomographic image inside the body cavity.

Methods that have been disclosed for scanning a light that are used by an OCT probe include a radial scanning method that rotates a fiber, a radial scanning method that rotates a deflection element that is provided at a distal end of a probe (PTL 2), a linear scanning method that causes a fiber to swing to the left and right using electromagnetic induction or the like (PTL 3), and a linear scanning method in which a small mirror is arranged inside the OCT probe (PTL 4). A two-dimensional scanning method that scans in a rotating direction and a direction perpendicular thereto while rotating has also been disclosed.

According to the conventional OCT apparatus, because the OCT probe is flexible and has an approximately cylindrical shape, it is difficult to stably maintain an observation position such as a position at which the OCT probe contacts living tissue or at which the OCT probe is separated by a certain distance from the living tissue for a period of time required for observation.

On the other hand, an endoscope that is equipped with a balloon, and an overtube for an endoscope have also been disclosed (PTL 5). This is a structure that is convenient for fixing an endoscope inside a digestive tract. However, according to this structure, there is the disadvantage that when an OCT probe is caused to protrude from a forceps opening of this kind of endoscope that is equipped with a balloon, the distance between the OCT probe and a wall surface is too great.

An OCT probe that is provided with a balloon has also been disclosed (PTL 6). Although the operability of the OCT probe unit is good, the OCT probe does not combine well with a normal endoscope, and it is difficult to contact the OCT probe against an affected area through a forceps channel of the endoscope.

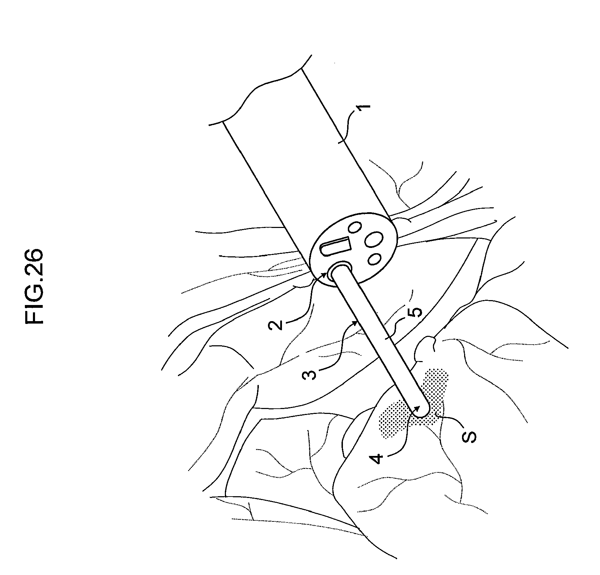

Figure 26 is an example of a view that illustrates a manner in which a tomographic image is obtained using an OCT probe that is led out from a distal end opening of a forceps channel of an endoscope. As shown in Figure 26, according to the OCT measurement method, a distal end portion 4 of an insertion portion of an OCT probe 3 that is caused to protrude from a distal end opening 2 of a forceps channel of an endoscope 1 is brought close to a desired site of a measurement target S to obtain a tomographic image.

In this case, for example, the elongated OCT probe 3 that is caused to protrude from the distal end opening 2 has insufficient rigidity because the sheath of the OCT probe 3 is flexible. Consequently, because the protruding portion of the OCT probe 3 easily bends in a manner in which the distal end opening 2 acts as a supporting point, and furthermore, due to the folded structure of tissue inside a body cavity or vibratile movements or the like, it is difficult to carry out an observation while maintaining a state in which the outer surface of a sheath 5 of the OCT probe 3 contacts against the surface of the measurement target S, or while maintaining the outer surface of the sheath 5 of the OCT probe 3 at a position that is further separated from the surface of the measurement target S relative to the aforementioned state.

Therefore, technology has been proposed that enables observation in a condition in which an optical probe is positioned at a fixed distance from living tissue (PTL 7).

### Citation List

### Patent Literature

PTL 1: Japanese Patent Application Laid-Open No. 6-165784
PTL 2: National Publication of International Patent Application No. 2000-503237
PTL 3: U.S. Patent No. 6950692
PTL 4: Japanese Patent Application Laid-Open No. 2004-229963
PTL 5: Japanese Patent Application Laid-Open No. 2005-168990
PTL 6: Japanese Patent Application Laid-Open No. 2007-75403
PTL 7: Japanese Patent Application Laid-Open No. 2002-263055

### Summary of Invention

### Technical Problem

However, according to the above described PTL 4, although a cap is provided at a distal end of the endoscope in order to position the optical probe at a fixed distance from the living tissue (measurement target S), with this configuration, not only is the distance between the optical probe and the living tissue (measurement target S) regulated by the opening position in the distal end face of the endoscope of the forceps channel, through which the optical probe is inserted, but it is also not possible to contact the optical probe against the living tissue (measurement target S). Furthermore, according to the technology described in PTL 4, there is also a risk that the cap will fall down from the distal end of the endoscope during observation (measurement).

The present invention has been made in view of the above described circumstances, and an object of the invention is to provide an optical probe and an optical observation device which can stably maintain a desired observation position, condense a measuring light onto a measurement target, and obtain structural information regarding the measurement target.

### Solution to Problem

To achieve the above object, an optical probe according to a first aspect of the present invention includes: a tubular sheath having a distal end that is closed; a light guiding means provided inside the sheath, and arranged along a longitudinal axis of the sheath; and a light condensing means that is provided on an emitting end side of the light guiding means, and that deflects an optical path of a light that is emitted from the emitting end so as to cause the light to converge on a measurement target; wherein the optical probe scans a convergent light that is emitted from the light condensing means on a plane that is substantially orthogonal to an optical axis direction of the convergent light, and guides a light from the measurement target to the light guiding means via the light condensing means, the optical probe further including a cylindrical tube member having an outer circumferential wall that is integrally joined to an outer wall of the sheath along a direction of the longitudinal axis.

According to the optical probe of the first aspect, by means of the cylindrical tube member that has an outer circumferential wall that is integrally joined to the outer wall of the sheath along the longitudinal axis direction, it is possible to stably maintain a desired observation position and condense a measuring light onto a measurement target to obtain structural information regarding the measurement target.

An optical probe according to a second aspect of the present invention is in accordance with the optical probe of the first aspect, wherein the tube member is an outer cylinder that has two ends that are open, and into which at least an endoscope insertion portion can be inserted.

An optical probe according to a third aspect of the present invention is in accordance with the optical probe of the first or second aspect, wherein an inner surface shape of a cross section of the sheath that is orthogonal to the longitudinal axis of the sheath is a substantially trapezoidal shape.

An optical probe according to a fourth aspect of the present invention is in accordance with the optical probe of the first or second aspect, wherein an inner surface shape of a cross section of the sheath that is orthogonal to the longitudinal axis of the sheath is a substantially semicircular shape.

An optical probe according to a fifth aspect of the present invention is in accordance with the optical probe of any one of the first to fourth aspects, wherein at least the light condensing means is configured to be movable within a plane that is orthogonal to the longitudinal axis of the sheath inside the sheath.

An optical probe according to a sixth aspect of the present invention is in accordance with the optical probe of any one of the first to fifth aspects, wherein the sheath has, at least at a distal end position of a side surface that faces an outer wall that is joined to the tube member, a translucent optical opening that transmits a light emitted from the light condensing means and a light from the measurement target.

An optical probe according to a seventh aspect of the present invention is in accordance with the optical probe of the sixth aspect, further including a balloon means that is expandable and contractible that is provided at an outer circumferential position facing an outer circumferential side that is joined to the sheath of the tube member, and a balloon control means that controls expansion and contraction of the balloon means.

An optical probe according to an eighth aspect of the present invention is in accordance with the optical probe of the sixth aspect, wherein the sheath includes a gap regulating means that is provided in a vicinity of the distal end position and that regulates a gap between an outer surface of the optical opening and a surface of the measurement target so that the gap is a predetermined distance.

An optical probe according to a ninth aspect of the present invention is in accordance with the optical probe of the eighth aspect, wherein the gap regulating means is a protrusion that is provided at a side surface position that is adjacent to the optical opening.

An optical probe according to a tenth aspect of the present invention is in accordance with the optical probe of the ninth aspect, wherein the protrusion is provided at front and rear of the optical opening (on a distal end side and a proximal end side of the sheath with respect to the opening) along the longitudinal axis of the sheath.

An optical probe according to an eleventh aspect of the present invention is in accordance with the optical probe of the seventh aspect, further including a gap regulating means that is provided in a vicinity of the distal end position and that regulates a gap between an outer surface of the optical opening and a surface of the measurement target so that the gap is a predetermined distance.

An optical probe according to a twelfth aspect of the present invention is in accordance with the optical probe of the eleventh aspect, wherein the gap regulating means is a protrusion that is provided at a side surface position that is adjacent to the optical opening.

An optical probe according to a thirteenth aspect of the present invention is in accordance with the optical probe of the twelfth aspect, wherein the protrusion is provided at front and rear of the optical opening along the longitudinal axis of the sheath.

An optical probe according to a fourteenth aspect of the present invention is in accordance with the optical probe of the twelfth or thirteenth aspect, wherein the protrusion is a balloon member that is expandable and contractible.

An optical probe according to a fifteenth aspect of the present invention is in accordance with the optical probe of the fourteenth aspect, wherein the balloon member is formed integrally with the balloon means.

An optical probe according to a sixteenth aspect of the present invention is in accordance with the optical probe of the fourteenth or fifteenth aspect, wherein the balloon control means further controls expansion and contraction of the balloon member.

An optical probe according to a seventeenth aspect of the present invention is in accordance with the optical probe of the eighth or eleventh aspect, wherein the gap regulating means is a concavity that is formed at a position on a distal end side surface of the sheath, and the optical opening is provided inside the concavity.

An optical probe according to an eighteenth aspect of the present invention is in accordance with the optical probe of any one of the first to seventeenth aspects, wherein the closed distal end of the sheath is formed in a tapered shape.

An optical observation device according to a nineteenth aspect of the present invention includes: an optical tomographic imaging apparatus that supplies a measuring light that is emitted to a measurement target inside a body cavity, and generates a tomographic image of the measurement target based on a light from the measurement target; and an optical probe that guides the measuring light to the measurement target, and guides a light from the measurement target to the optical tomographic imaging apparatus; the optical probe including: a tubular sheath having a distal end that is closed, a light guiding means that is provided inside the sheath and that is arranged along a longitudinal axis of the sheath, a light condensing means that is provided on an emitting end side of the light guiding means and that deflects an optical path of a light emitted from the emitting end to cause the light to converge on a measurement target, and a cylindrical tube member through which at least an elongated endoscope insertion portion having flexibility can be inserted and which has an outer circumferential wall that is integrally joined to an outer wall of the sheath along a direction of the longitudinal axis; wherein the optical probe scans a convergent light emitted from the light condensing means on a plane that is substantially orthogonal to an optical axis direction of the convergent light, and guides a light from the measurement target to the light guiding means via the light condensing means.

According to the optical observation device of the nineteenth aspect, by means of the cylindrical tube member that has an outer circumferential wall that is integrally joined to the outer wall of the sheath along the longitudinal axis direction, it is possible to stably maintain a desired observation position and condense a measuring light onto a measurement target to obtain structural information regarding the measurement target.

An optical observation device according to a twentieth aspect of the present invention is in accordance with the optical observation device of the nineteenth aspect, which can be configured to further include a balloon means that is expandable and contractible that is provided at an outer circumferential position facing an outer circumferential wall that is joined to the sheath of the tube member, and a balloon control portion that controls expansion and contraction of the balloon means.

An optical observation device according to a twenty-first aspect of the present invention is in accordance with the optical observation device of the nineteenth or twentieth aspect, further including: a translucent optical opening that is provided at least at a distal end position of a side surface that faces an outer wall that is joined to the tube member of the sheath, and that transmits a light emitted from the light condensing means and a light from the measurement target; and a gap regulating means that is provided in a vicinity of the distal end position and that regulates a gap between an outer surface of the optical opening and a surface of the measurement target so that the gap is a predetermined distance.

An optical observation device according to a twenty-second aspect of the present invention includes: an optical tomographic imaging apparatus that supplies a measuring light that is emitted to a measurement target inside a body cavity, and generates a tomographic image of the measurement target based on a light from the measurement target; an optical probe that guides the measuring light to the measurement target, and guides a light from the measurement target to the optical tomographic imaging apparatus; a light source means that supplies an illuminating light that is emitted to the measurement target; an endoscope that includes an elongated insertion portion that has flexibility, and that picks up an image of the measurement target to which the illuminating light is emitted; and an endoscope processor that carries out signal processing with respect to an image pickup signal from the endoscope, and generates an endoscopic image of the measurement target; the optical probe including: a tubular sheath having a distal end that is closed; a light guiding means that is provided inside the sheath, and that is arranged along a longitudinal axis of the sheath; a light condensing means that is provided on an emitting end side of the light guiding means, and that deflects an optical path of a light emitted from the emitting end to cause the light to converge on a measurement target; and a cylindrical tube member through which at least the insertion portion of the endoscope can be inserted, and which has an outer circumferential wall that is integrally joined to an outer wall of the sheath along a direction of the longitudinal axis; wherein the optical probe scans a convergent light that is emitted from the light condensing means on a plane that is substantially orthogonal to an optical axis direction of the convergent light, and guides a light from the measurement target to the light guiding means via the light condensing means.

According to the optical observation device of the twenty-second aspect, by means of the cylindrical tube member that has an outer circumferential wall that is integrally joined to the outer wall of the sheath along the longitudinal axis direction, it is possible to stably maintain a desired observation position and condense a measuring light onto a measurement target to obtain structural information regarding the measurement target.

An optical observation device according to a twenty-third aspect of the present invention is in accordance with the optical observation device of the twenty-second aspect, further including: a balloon means that is expandable and contractible that is provided at an outer circumferential position that faces an outer circumferential wall that is joined to the sheath of the tube member; and a balloon control portion that controls expansion and contraction of the balloon means.

An optical observation device according to a twenty-fourth aspect of the present invention is in accordance with the optical observation device according to the twenty-second or twenty-third aspect, further including: a translucent optical opening that is provided at least at a distal end position of a side surface that faces an outer wall that is joined to the tube member of the sheath, and that transmits a light emitted from the light condensing means and light from the measurement target; and a gap regulating means that is provided in a vicinity of the distal end position and that regulates a gap between an outer surface of the optical opening and a surface of the measurement target so that the gap is a predetermined distance.

### Advantageous Effects of Invention

As described above, according to the present invention there is the advantageous effect that it is possible to stably maintain a desired observation position and condense a measuring light onto a measurement target to obtain structural information regarding the measurement target.

### Brief Description of Drawings

Figure 1 is an external view of a diagnostic imaging apparatus according to a first embodiment;
Figure 2 is a view that illustrates a configuration of an OCT probe shown in Figure 1;
Figure 3 is a cross-sectional view that illustrates a cross section along a line A-A in Figure 2;
Figure 4 is a cross-sectional view that illustrates a first modification example of the cross section along the line A-A in Figure 2;
Figure 5 is a cross-sectional view that illustrates a second modification example of the cross section along the line A-A in Figure 2;
Figure 6 is a cross-sectional view that illustrates a cross section of a distal end of the OCT probe and an insertion portion of an endoscope shown in Figure 1;
Figure 7 is a view that illustrates a first modification example of the OCT probe shown in Figure 6;
Figure 8 is a view that illustrates a second modification example of the OCT probe shown in Figure 6;
Figure 9 is a view that illustrates a configuration of an OCT probe according to a second embodiment;
Figure 10 is a first view that describes an action of the OCT probe shown in Figure 9;
Figure 11 is a second view that describes an action of the OCT probe shown in Figure 9;
Figure 12 is a view that illustrates a distal end configuration of an OCT probe according to a third embodiment;
Figure 13 is a view that illustrates a positional relationship between protrusions and an optical opening shown in Figure 12;
Figure 14 is a view that illustrates a modification example of the positional relationship between the protrusions and the optical opening shown in Figure 13;
Figure 15 is a view that illustrates a first modification example of the distal end configuration of the OCT probe shown in Figure 12;
Figure 16 is a view that illustrates a second modification example of the distal end configuration of the OCT probe shown in Figure 12;
Figure 17 is a view that illustrates a third modification example of the distal end configuration of the OCT probe shown in Figure 12;
Figure 18 is a view that illustrates a positional relationship between protrusions and an optical opening shown in Figure 17;
Figure 19 is a view that illustrates a distal end configuration of an OCT probe according to a fourth embodiment;
Figure 20 is a view that illustrates a positional relationship between a concavity and an optical opening shown in Figure 19;
Figure 21 is a view that illustrates a configuration of a modification example of a tube member according to the first to fourth embodiments;
Figure 22 is a view that illustrates a configuration of a first modification example of the OCT probe according to the first to fourth embodiments;
Figure 23 is a view that illustrates a configuration of a second modification example of the OCT probe according to the first to fourth embodiments;
Figure 24 is a view that shows side surface of the OCT probe shown in Figure 23;
Figure 25 is a view that illustrates a configuration of a third modification example of the OCT probe according to the first to fourth embodiments;
Figure 26 is a view that illustrates a manner in which a tomographic image is obtained using an OCT probe that is led out from a distal end opening of a forceps channel of an endoscope.

### Description of Embodiments

Modes for carrying out the present invention are described hereunder with reference to the attached drawings.

### First Embodiment:

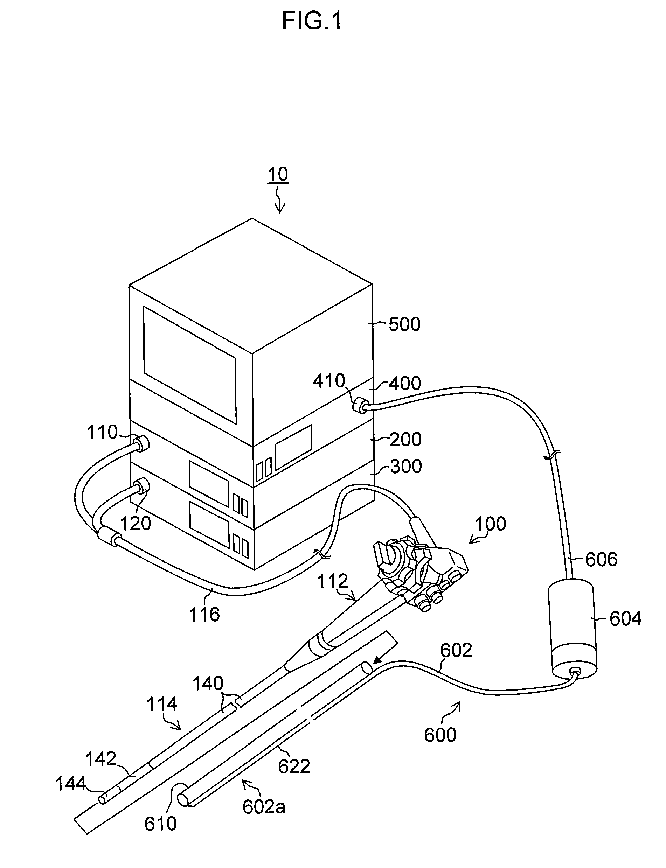

Figure 1 is an example of an external view that illustrates a diagnostic imaging apparatus according to a first embodiment. As shown in Figure 1, a diagnostic imaging apparatus 10 as an optical observation device of the first embodiment is mainly constituted by an endoscope 100, an endoscope processor 200, a light source apparatus 300 as a light source means, an OCT processor 400 as an optical tomographic imaging apparatus, and a monitor device 500. The endoscope processor 200 may be configured so as to incorporate the light source apparatus 300.

The endoscope 100 includes a hand operation portion 112 and an insertion portion 114 that is connected in series to the hand operation portion 112. A physician grasps and operates the hand operation portion 112 and inserts the insertion portion 114 into the body of a subject to observe the inside of the body.

A universal cable 116 is connected to the hand operation portion 112. An LG (light guide) connector 120 is provided at a distal end of the universal cable 116. By detachably connecting the LG connector 120 to the light source apparatus 300, an illuminating light can be fed to an illumination optical system (not shown) that is arranged in a distal end portion of the insertion portion 114. An electrical connector 110 is also connected to the LG connector 120 via the universal cable 116. The electrical connector 110 is detachably connected to the endoscope processor 200. Thus, data of an observed image that is obtained with the endoscope 100 is output to the endoscope processor 200, and an image is displayed on the monitor device 500 connected to the endoscope processor 200.

An air/water feeding button, a suction button, a shutter button, a function switching button, a pair of angle knobs, and a pair of lock levers are provided in the hand operation portion 112. However, further description of these members is omitted hereunder.

The OCT probe 600 includes an insertion portion 602, an operation portion 604 with which the physician operates the OCT probe 600, and a cable 606 that is connected to the OCT processor 400 via a connector 410.

A cylindrical tube member 610 is provided in the insertion portion 602 of the OCT probe 600. The cylindrical tube member 610 is integrally formed on an outer circumference of a sheath 602a of the insertion portion 602 along a longitudinal axis direction thereof. Both ends of the tube member 610 are open, and the insertion portion 114 of the endoscope 100 can be inserted therethrough. The sheath 602a and the tube member 610 are each constituted by a flexible tubular member.

The insertion portion 114 of the endoscope 100 includes, in order from the hand operation portion 112 side, a flexible portion 140, a bending portion 142, and a distal end portion 144. An observation optical system and an illumination optical system and the like that are described later are provided in the distal end portion 144.

The configurations of the endoscope processor and the light source apparatus are known, and hence a description thereof is omitted herein. The OCT processor 400 is a processor that utilizes, for example, an SS-OCT (Swept Source OCT) measurement method, and a description thereof is omitted herein since the details are known. In this connection, for example, a known TD-OCT (Time Domain OCT) measurement method or SD-OCT (Spectral Domain OCT) measurement method may be used as the OCT measurement method.

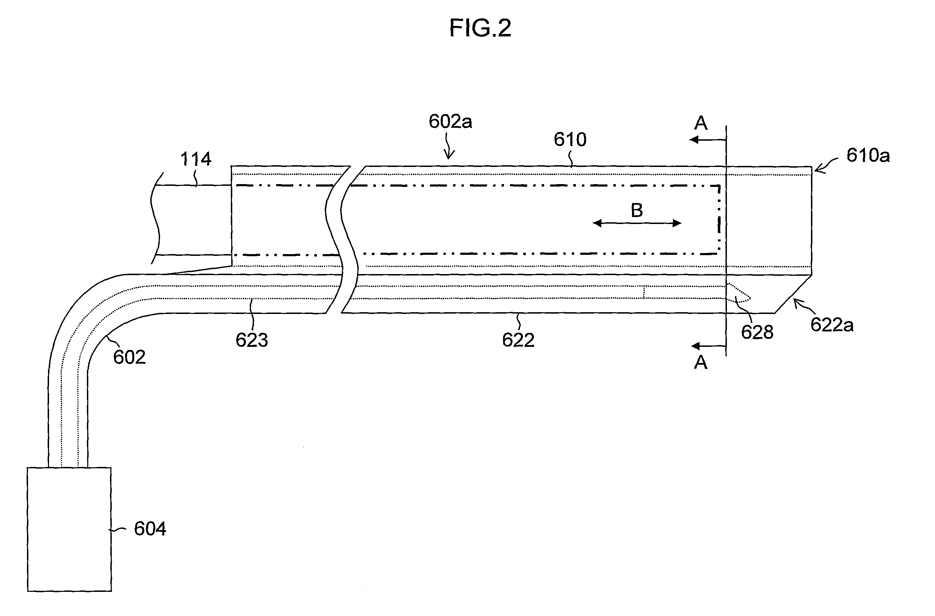

Figure 2 is a view that illustrates a configuration of the OCT probe shown in Figure 1. As shown in Figure 2, in the insertion portion 602 of the OCT probe 600, a sheath of the insertion portion 602 is constituted by the double sheath 602a that includes a tubular OCT sheath portion 622 through which an optical fiber 623 as a light guiding means for OCT measurement is inserted, and the cylindrical tube member 610 for inserting the insertion portion 114 of the endoscope 100 that is integrally formed on an outer circumference of the insertion portion 602 along a direction of the longitudinal axis of the insertion portion 602 from a predetermined position on a proximal end side of the insertion portion 602 to the distal end thereof. An optical lens 628 as a light condensing means is installed at the distal end of the optical fiber 623. The optical lens 628 is constituted by a hemispherical lens. A distal end 622a of the OCT sheath portion 622 has a closed (sealed) structure, and is formed as a tapered structure so that the distal end 622a does not become caught in tissue or the like when the insertion portion 602 is inserted into a body. The two ends of the tube member 610 are open, and the insertion portion 114 of the endoscope 100 can advance and retract inside the tube member 610 in the direction (longitudinal axis direction) indicated by arrow B. The insertion portion 114 of the endoscope 100 can be caused to protrude further than a distal end 610a of the tube member 610.

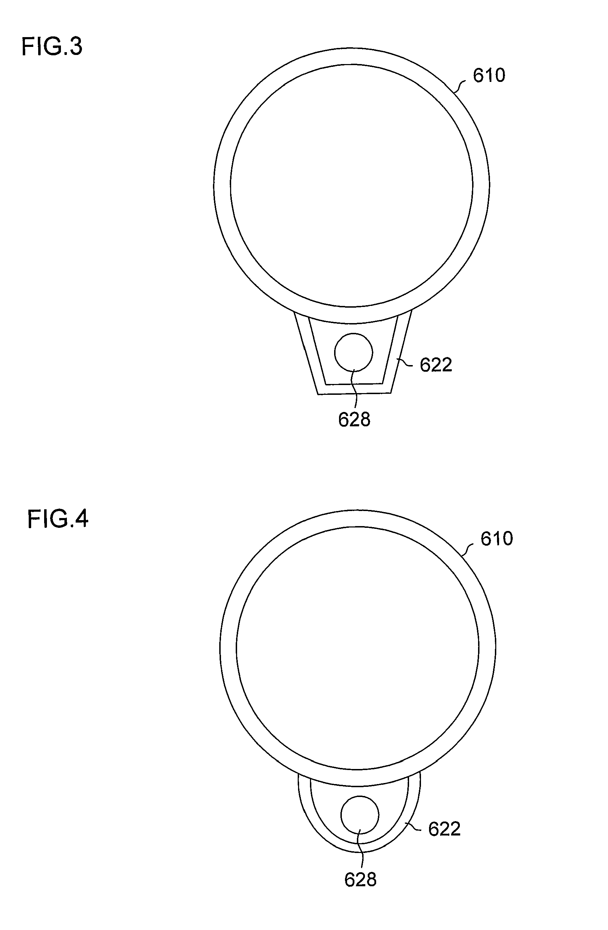

Figure 3 is a cross-sectional view that illustrates a cross section along line A-A in Figure 2. As shown in Figure 3, on a plane that is orthogonal to the longitudinal axis of the insertion portion 602, a cross section of the OCT sheath portion 622 is formed in a substantially trapezoidal shape and a cross section of the tube member 610 is formed in a substantially circular shape. By making the bottom surface of the OCT sheath portion 622 a surface flat in this manner, contact of the OCT sheath portion 622 against a measurement target can be stabilized.

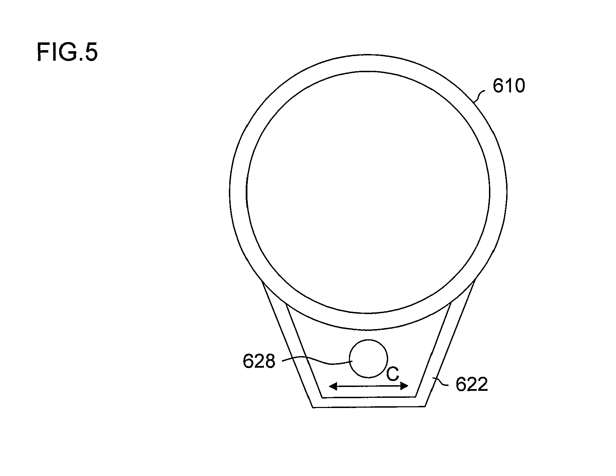

Figure 4 is a cross-sectional view that illustrates a first modification example of the cross section along the line A-A in Figure 2. Figure 5 is a cross-sectional view that illustrates a second modification example of the cross section along the line A-A in Figure 2. As shown in Figure 4, the cross section of the OCT sheath portion 622 may be formed in a substantially semicircular shape on the plane orthogonal to the longitudinal axis of the insertion portion 602. Further, as shown in Figure 5, a configuration may be adopted in which the cross section of the OCT sheath portion 622 is formed in a substantially trapezoidal shape on the plane orthogonal to the longitudinal axis of the insertion portion 602, and the optical lens 628 is movable in a direction indicated by arrow C (at least a direction parallel to the longitudinal axis on a plane orthogonal to the longitudinal axis of the insertion portion 602) inside the OCT sheath portion 622.

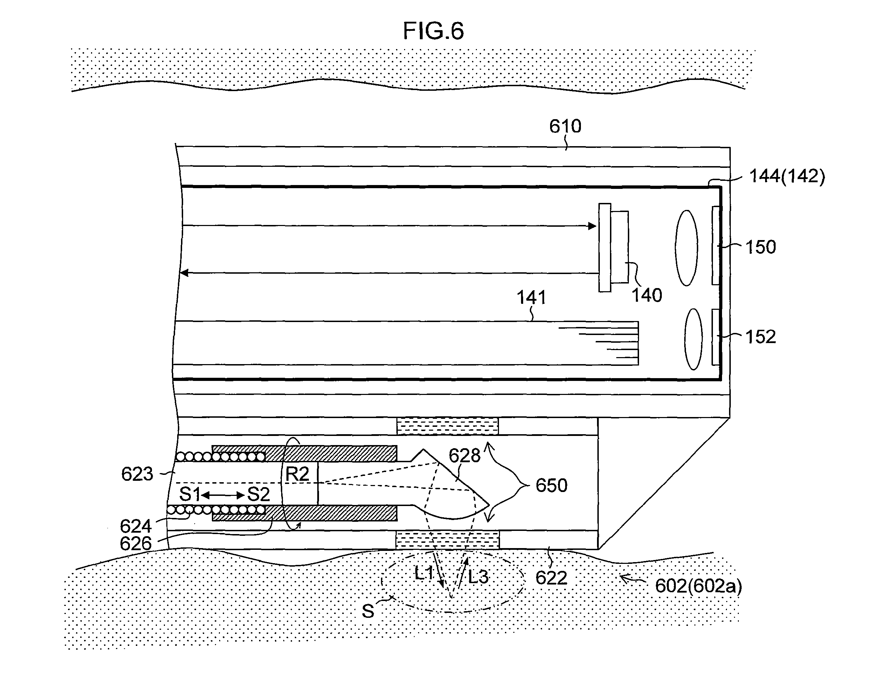

Figure 6 is a cross-sectional view that illustrates a cross section of a distal end of the OCT probe and the insertion portion of the endoscope shown in Figure 1. As shown in Figure 6, an observation optical system 150, an illumination optical system 152 and the like are provided in the distal end portion 144 of the insertion portion 114 of the endoscope 100 that is inserted through the tube member 610. A CCD image sensor 143 that is a solid-state image pickup device is arranged inside the distal end portion 144 at the rear of the observation optical system 150.

An observation image that is captured by the observation optical system 150 undergoes image formation on a light receiving surface of the CCD image sensor 143 and is converted into an electrical signal. The electrical signal is output to the endoscope processor 200, and is converted into a video signal. As a result, an observed image is displayed on the monitor device 500 that is connected to the endoscope processor 200 (see Figure 1).

The illumination optical system 152 is provided adjacent to the observation optical system 150, and if needed may be arranged on both sides of the observation optical system 150. An emitting end of a light guide 141 is arranged at the rear of the illumination optical system 152. The light guide 141 is inserted through the insertion portion 114, the hand operation portion 112, and the universal cable 116, and an incident end of the light guide 141 is arranged inside the LG connector 120. Accordingly, by connecting the LG connector 120 to the light source apparatus 300, an illuminating light that is emitted from the light source apparatus 300 is transmitted to the illumination optical system 152 through the light guide 141, and is emitted from the illumination optical system onto an observation range in front of the illumination optical system 152 (see Figure 1).

In the OCT sheath portion 622, at least one part of a side surface on a distal end side through which a measuring light L1 and a reflected light L3 pass via the optical lens 628 includes an optical opening 650 that is formed by a member (translucent member) that transmits light across the entire circumference thereof.

The optical fiber 623 is a linear member that is housed inside the OCT sheath portion 622 along the longitudinal axis thereof. The optical fiber 623 guides the measuring light L1 emitted from the OCT processor 400 as far as the optical lens 628. The optical fiber 623 also guides the reflected light L3 from a measurement target S that is acquired at the optical lens 628 as light that is reflected when the measuring light L1 is emitted to the measurement target S, to the OCT processor 400.

The optical fiber 623 is connected to a rotary joint (not illustrated) or the like inside a rotational driving portion (not illustrated) provided in the operation portion 604 of the OCT probe 600, and is optically connected to the OCT processor 400. The optical fiber 623 is arranged in a freely rotatable state with respect to the OCT sheath portion 622.

A flexible shaft 624 is fixed to the outer circumference of the optical fiber 623. The optical fiber 623 and the flexible shaft 624 are connected to the rotational driving portion (not illustrated), and the rotational driving portion (not illustrated) causes the optical lens 628 to rotate in the direction of arrow R2 with respect to the OCT sheath portion 622.

The optical lens 628 is arranged at the distal end of the optical fiber 623, and a distal end portion thereof is formed in a substantially spherical shape in order to condense the measuring light L1 emitted from the optical fiber 623 onto the measurement target S.

The optical lens 628 irradiates the measurement target S with the measuring light L1 emitted from the optical fiber 623, condenses the reflected light L3 from the measurement target S, and causes the condensed reflected light L3 to be incident on the optical fiber 623.

A fixing member 626 is arranged at an outer circumference of a connecting portion between the optical fiber 623 and the optical lens 628. The fixing member 626 fixes the optical lens 628 to the distal end of the optical fiber 623.

The optical fiber 623, the flexible shaft 624, the fixing member 626, and the optical lens 628 are configured to be movable in the direction of an arrow S1 (direction of the proximal end of the OCT sheath portion 622) and an arrow S2 (direction of the distal end of the OCT sheath portion 622) inside the OCT sheath portion 622 by means of an advancing and retracting mechanism (not illustrated) of the rotational driving portion.

In the OCT probe 600 configured as described above, by rotating the optical fiber 623 and the flexible shaft 624 in the direction of arrow R2 in Figure 6 by means of the rotational driving portion, the measuring light L1 emitted from the optical lens 628 is emitted to the measurement target S while being scanned in the arrow R2 direction (circumferential direction of the OCT sheath portion 622), and the returning light L3 is acquired.

According to the present embodiment, as shown in Figure 6, first, the physician inserts the insertion portion 114 of the endoscope 100 into the tube member 610 of the double sheath 602a. Subsequently, while viewing an image obtained with the endoscope 100, the physician inserts the double sheath 602a, through which the insertion portion 114 of the endoscope 100 has been inserted, into the body (into a lumen) of the patient. When the physician finds an affected area as the measurement target S in the body (in the lumen) based on the endoscopic image, the physician rotates the double sheath 602a to perform an adjustment so that the OCT sheath portion 622 side faces the direction of the measurement target S, and causes the optical opening 650 of the OCT sheath portion 622 to contact against the measurement target S. In this state, the physician moves the optical fiber 623 and the optical lens 628 that are inside the OCT sheath portion 622 in the longitudinal axis direction while rotating the optical fiber 623 and the optical lens 628, to thereby perform two-dimensional scanning. Since information regarding the depth direction is obtained by the OCT measurement, the result of the measurement process is that data regarding the three-dimensional structure of the measurement target S is obtained.

As described above, according to the present embodiment, since the double sheath 602a is constituted by the tube member 610 and the OCT sheath portion 622, the overall rigidity of the double sheath 602a is increased by the tube member 610. Because of the overall rigidity of the double sheath 602a, it is possible to stably contact the optical opening 650 of the OCT sheath portion 622 against the measurement target S.

More specifically, the OCT probe 600 stably maintains a contact position with the measurement target S that is a desired observation position, condenses a measuring light onto the measurement target S, and guides a light from the measurement target S to the OCT processor 400. It is thus possible to acquire data regarding the three-dimensional structure of the measurement target S, and obtain an optical tomographic image of the measurement target S.

Moreover, in a state in which the insertion portion 114 of the endoscope 100 is inserted through the tube member 610, that is, under endoscopic observation, the overall rigidity of the double sheath 602a is further increased in the OCT probe 600, and a contact position with the measurement target S can be maintained with even greater stability by utilizing a bending operation of the bending portion 142 of the insertion portion 114.

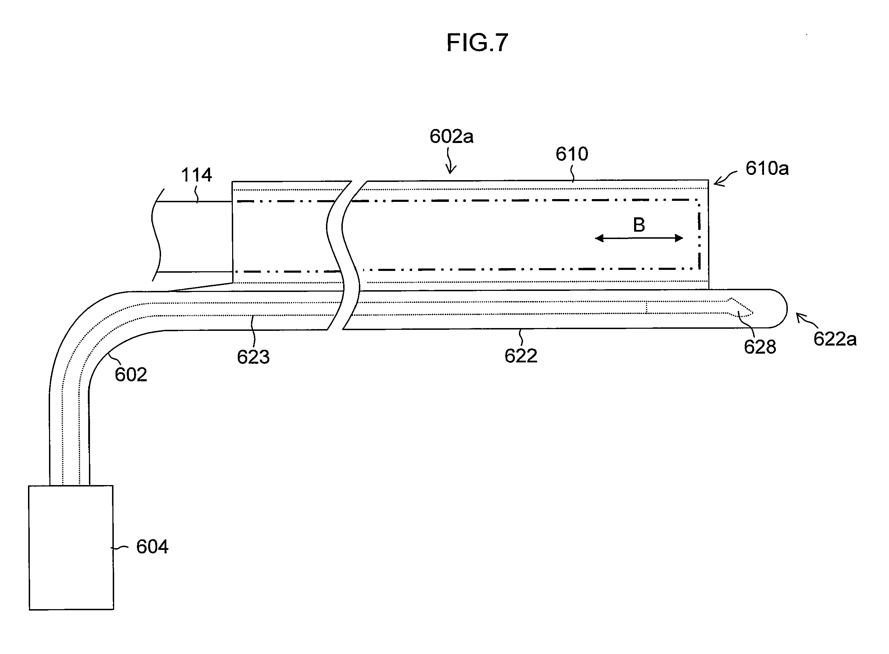

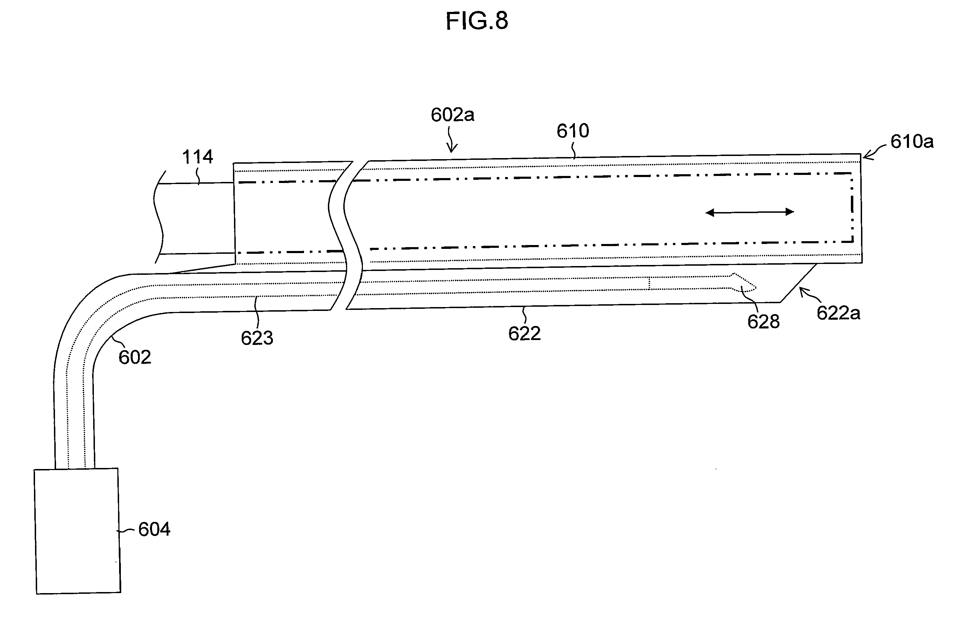

In this connection, although according to the present embodiment an example has been described in which a position of the distal end 622a of the OCT sheath portion 622 and a position of the distal end 610a of the tube member 610 are substantially the same as shown in Figure 6, an embodiment of the present invention is not limited thereto. Figure 7 is a view that illustrates a first modification example of the OCT probe shown in Figure 6, while Figure 8 is a view that illustrates a second modification example of the OCT probe shown in Figure 6. More specifically, as shown in Figure 7, the double sheath 602a may be configured so that the distal end 622a of the OCT sheath portion 622 is located at a position that is further on the distal end side than the distal end 610a of the tube member 610. Alternatively, as shown in Figure 8, the double sheath 602a may be configured so that the distal end 622a of the OCT sheath portion 622 is located at a position that is further on the proximal end side than the distal end 610a of the tube member 610.

### Second Embodiment:

The second embodiment is almost the same as the first embodiment. Hence only differences in the configuration thereof relative to the first embodiment are described hereafter, and like components are designated by like reference symbols and a description of such components is omitted.

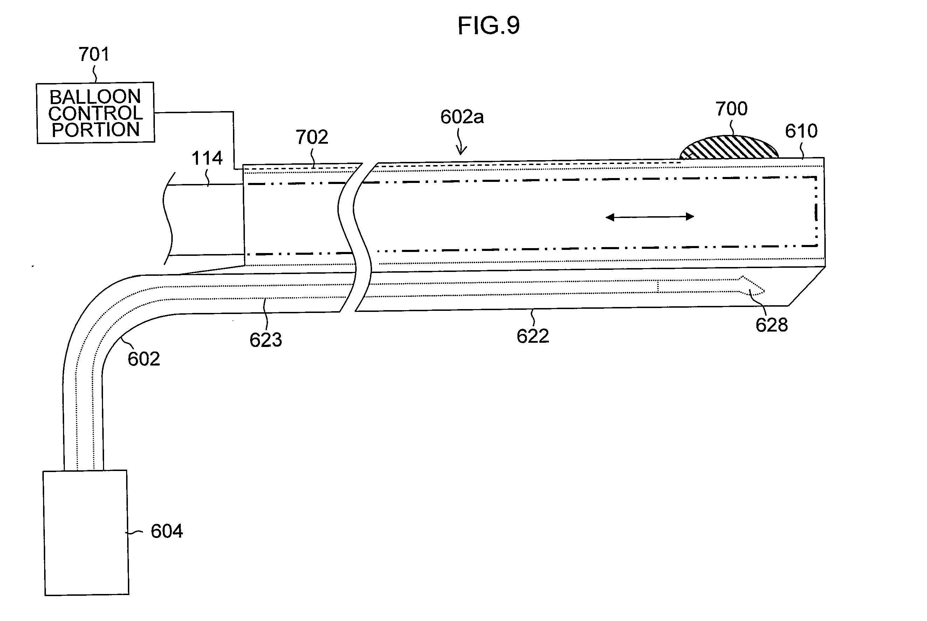

Figure 9 is a view that illustrates the configuration of an OCT probe according to the second embodiment. According to the present embodiment, as shown in Figure 9, a balloon 700 is provided on a side surface of the tube member 610 on a side that faces a position at which the OCT sheath portion 622 contacts against the measurement target S. A balloon control portion 701 for expanding/contracting the balloon 700 is connected to the balloon 700 via an air-supply path 702 provided inside the side surface of the tube member 610 along the longitudinal axis thereof. The remaining configuration is the same as in the first embodiment.

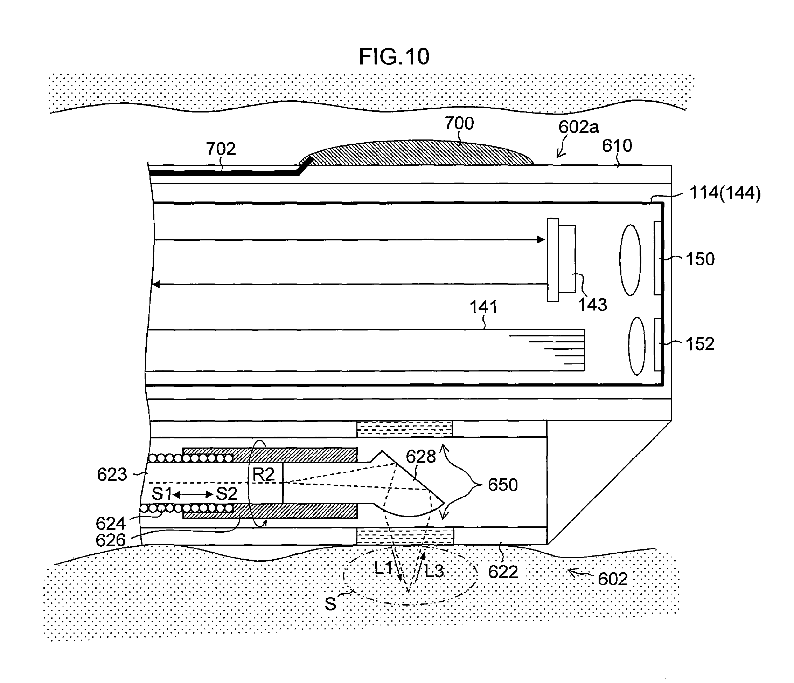

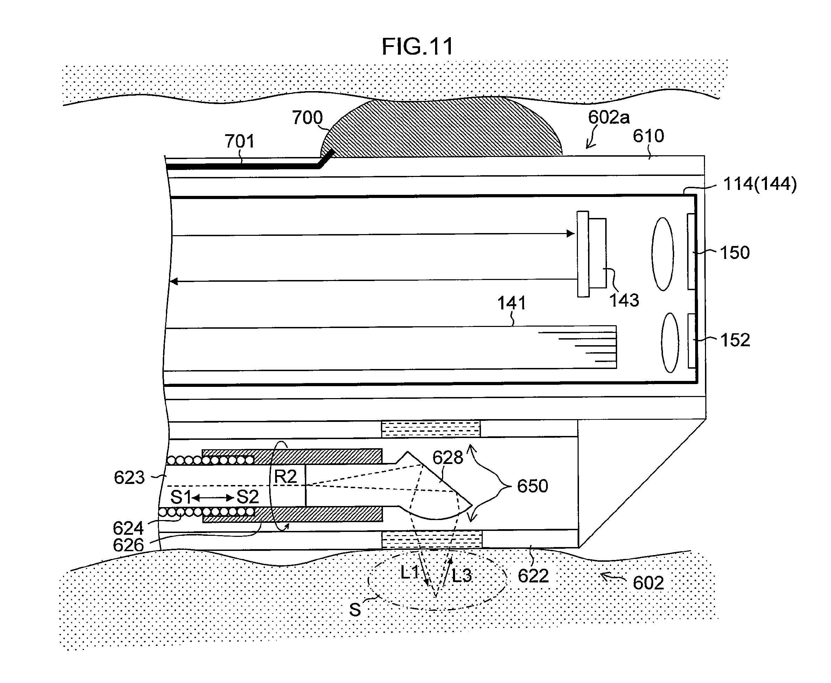

Figure 10 is a first view that describes an action of the OCT probe shown in Figure 9. Figure 11 is a second view that describes an action of the OCT probe shown in Figure 9.

As shown in Figure 10, similarly to the first embodiment, according to the present embodiment the physician first inserts the insertion portion 114 of the endoscope 100 inside the tube member 610 of the double sheath 602a. Subsequently, while viewing an image acquired with the endoscope 100, the physician inserts the double sheath 602a, through which the insertion portion 114 of the endoscope 100 has been inserted, into the body (into a lumen) of the patient. When the physician finds an affected area as the measurement target S in the body (in the lumen) based on the endoscopic image, the physician rotates the double sheath 602a to perform an adjustment so that the OCT sheath portion 622 side faces the direction of the measurement target S, and causes the optical opening 650 of the OCT sheath portion 622 to contact against the measurement target S.

According to the present embodiment, as shown in Figure 11, next the physician operates the balloon control portion 701 to inflate the balloon 700 and push the double sheath 602a against the inner wall of the lumen and thereby fix the portion of contact between the measurement target S and the optical opening 650. In this state, the physician moves the optical fiber 623 and the optical lens 628 that are inside the OCT sheath portion 622 in the longitudinal axis direction while rotating the optical fiber 623 and the optical lens 628, to thereby perform two-dimensional scanning. Since information regarding the depth direction is obtained by the OCT measurement, the result of the measurement process is that data regarding the three-dimensional structure of the measurement target S is obtained.

Thus, according to the present embodiment, in addition to the advantageous effects of the first embodiment, an advantageous effect is obtained such that, while the double sheath 602a is fixed at the measurement target S by means of the balloon 700, it is possible to exchange endoscopes within a range of endoscopes that can enter the tube member 610.

For example, after the position of the measurement target S has been identified using an endoscope that enables normal observation or special light observation when the double sheath 602a is inserted into the body of the patient, the physician can exchange the endoscope in question for an endoscope through which a treatment instrument can be inserted, thereby allowing the physician to perform treatment of the measurement target S.

Although not shown in the drawings, for example, the OCT probe 600 that has the double sheath 602a in which the inner diameter of the tube member 610 is, for example, 1.0 mm is used for a biopsy of the periphery of the lung (bronchial tubes). Subsequently, a thin endoscope 100 with a diameter of, for example, 0.8 mm is inserted into the tube member 610 having the inner diameter of 1.0 mm. The endoscope 100 is provided with an illumination light portion for fluorescence observation and an observation camera, and does not have a forceps opening. While carrying out observation using the endoscope 100, the physician inserts the endoscope 100 as far as the periphery of the lung (bronchial tubes). When the physician finds a location that resembles a lesion portion while also using fluorescence observation, the physician carries out OCT measurement at that location. The physician utilizes the OCT measurement to check for hypertrophy or alteration of the wall surface. By inflating the balloon 700, the physician can withdraw the endoscope 100 from the tube member 610 while retaining the double sheath 602a at the location in question. Subsequently, the physician passes a biopsy forceps through the tube member 610 in place of the endoscope 100, and performs a biopsy of the measurement target S for which OCT measurement has been performed. Thus, it is possible to perform a biopsy of the lesion portion with a high degree of accuracy. In this connection, the term "biopsy" refers to extracting part of the living body from the measurement target S (subject) and determining the presence or absence of a lesion or the like.

### Third Embodiment:

The third embodiment is almost the same as the first embodiment. Hence only differences in the configuration thereof relative to the first embodiment are described hereafter, and like components are designated by like reference symbols and a description of such components is omitted.

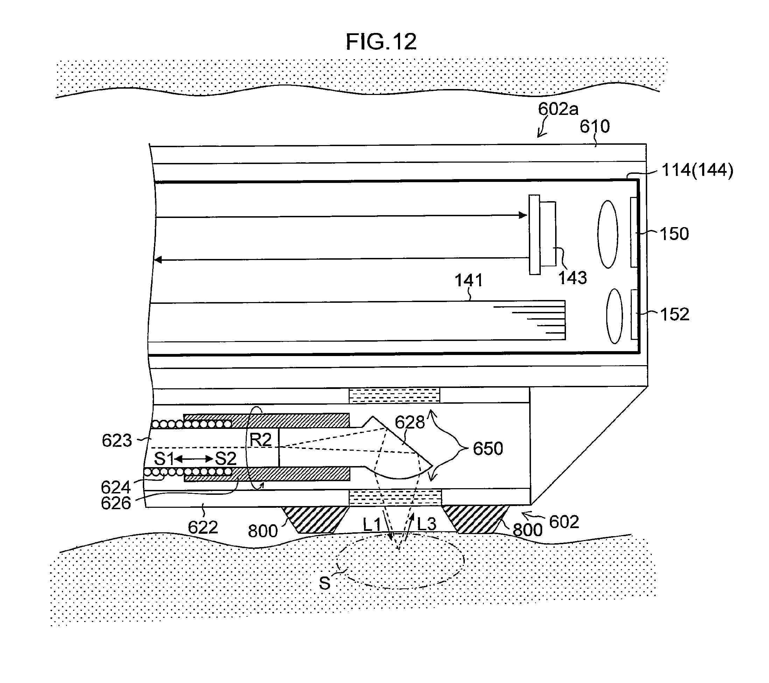

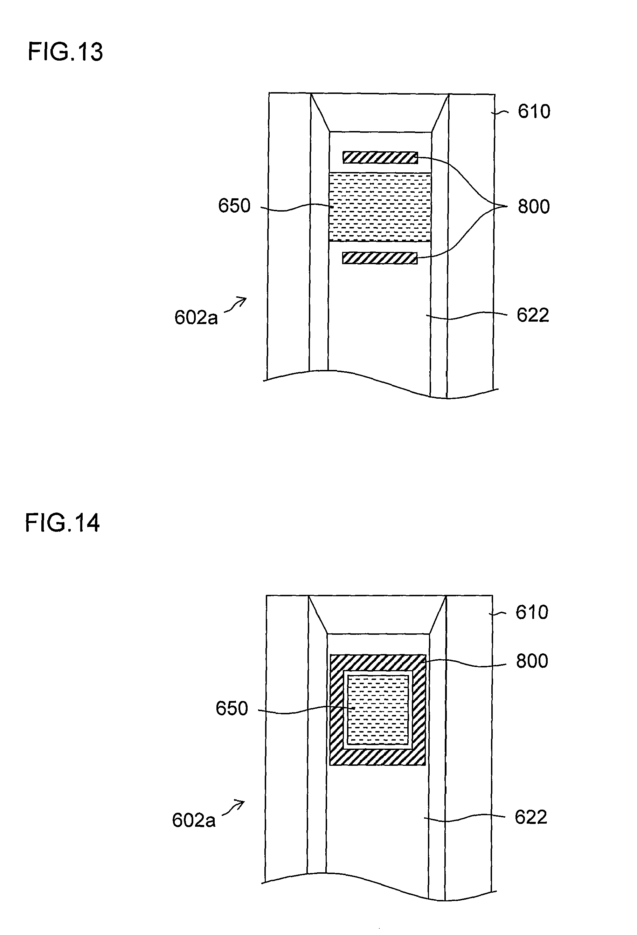

Figure 12 is a view that illustrates a distal end configuration of an OCT probe according to the third embodiment. Figure 13 is a view that illustrates a positional relationship between protrusions and an optical opening that are shown in Figure 12.

According to the present embodiment, as shown in Figure 12, protrusions 800 as a gap regulating means are formed at positions to the front and rear (a proximal end side position and a distal end side position: see Figure 13) of the optical opening 650 of the OCT sheath portion 622. The remaining configuration is the same as in the first embodiment.

As shown in Figure 12, similarly to the first embodiment, according to the present embodiment the physician first inserts the insertion portion 114 of the endoscope 100 into the tube member 610 of the double sheath 602a. Subsequently, while viewing an image obtained with the endoscope 100, the physician inserts the double sheath 602a, through which the insertion portion 114 of the endoscope 100 has been inserted, into the body (into a lumen) of the patient. When the physician finds an affected area as the measurement target S in the body (in the lumen) based on the endoscopic image, the physician rotates the double sheath 602a to perform an adjustment so as to make the OCT sheath portion 622 side face in the direction of the measurement target S, and presses the optical opening 650 of the OCT sheath portion 622 towards the measurement target S. Because of the existence of the protrusions 800, the optical opening 650 is poised over the measurement target S at a height that is equal to the height of the protrusions 800, and is thereby positioned at a desired height (for example, 1 mm). In this state (state in which the optical opening is at an observation position), the physician moves the optical fiber 623 and the optical lens 628 that are inside the OCT sheath portion 622 in the longitudinal axis direction while rotating the optical fiber 623 and the optical lens 628, to thereby perform two-dimensional scanning. Since information regarding the depth direction is obtained by the OCT measurement, the result of the measurement process is that data regarding the three-dimensional structure of the measurement target S is obtained.

Thus, according to the present embodiment, in addition to the advantageous effects of the first embodiment, an advantageous effect is obtained such that a distance between the optical opening 650 and the measurement target S can be regulated to a predetermined distance by the protrusions 800 as a gap regulating means, to thereby ensure a gap therebetween and enable measurement from a desired observation position. Consequently, in contrast to the first embodiment in which data regarding the three-dimensional structure of a deep layer portion of the measurement target S is acquired, according to the present embodiment, for example, data regarding the three-dimensional structure of a surface layer portion of the measurement target S can be acquired. More specifically, according to the present embodiment, measurement can be performed from a desired observation position based on the height of the protrusions 800 as a gap regulating means, and data regarding the three-dimensional structure of a layer portion of a desired depth of the measurement target S can be acquired.

Figure 14 is a view that illustrates a modification example of the positional relationship between the protrusions and the optical opening shown in Figure 13. According to the present embodiment, a configuration is adopted in which the protrusions 800 are provided at the front and rear of the optical opening 650 (see Figure 13). However, the present invention is not limited thereto and, as shown in Figure 14, the protrusion 800 may be formed so as to surround the optical opening 650.

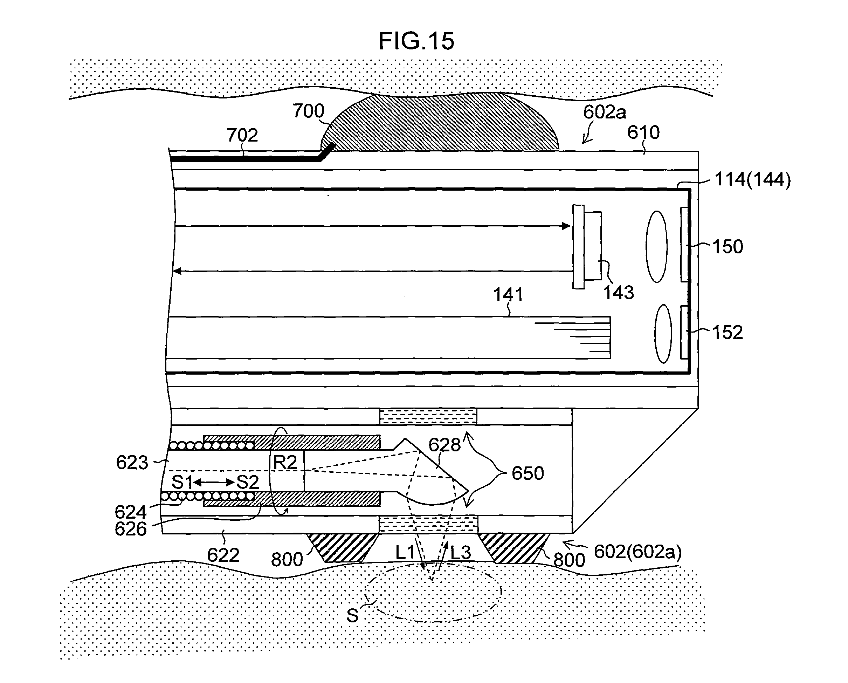

Figure 15 is a view that illustrates a first modification example of the distal end configuration of the OCT probe shown in Figure 12. According to the present embodiment also, as a first modification example, similarly to the second embodiment, the balloon 700 may be provided as shown in Figure 15.

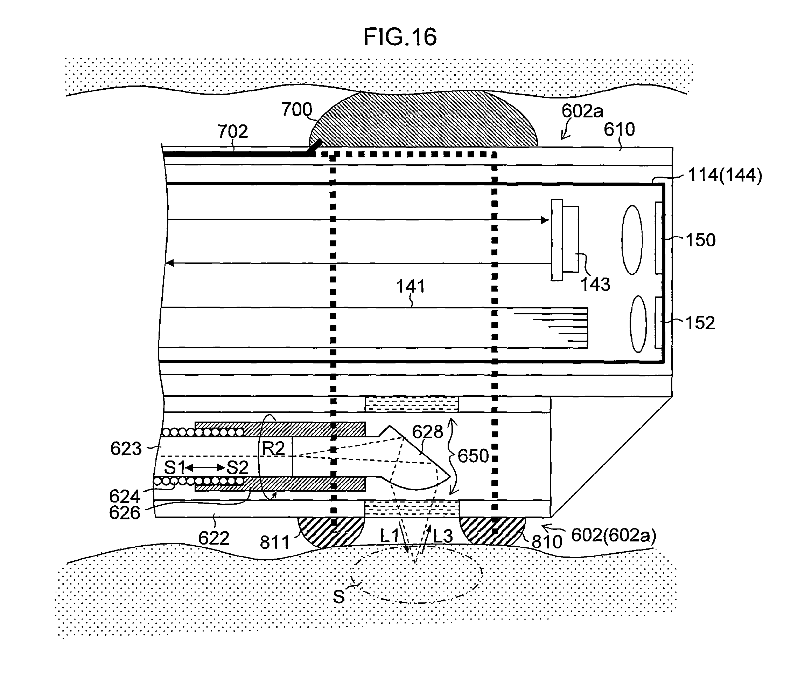

Figure 16 is a view that illustrates a second modification example of the distal end configuration of the OCT probe shown in Figure 12. According to the present embodiment, as a second modification example, as shown in Figure 16, in addition to the balloon 700, the protrusions 800 may also be constituted by balloons 810 and 811, and the balloon control portion 701 can be operated to inflate the balloons 700, 810, and 811 to fix the double sheath 602a. In this case, first, the balloon 700 and the first balloon 810 on the distal end side that is to the front of the measurement target S are inflated to fix the double sheath 602a. Next, the double sheath 602a is pushed into the measurement target S to firmly stretch out the vicinity of the measurement target S. When the vicinity of the measurement target S is stretched out, the second balloon 811 on the proximal end side is inflated and fixed. It is thus possible to prevent the measurement target S from entering the space between the balloons 810 and 811 in a case where the periphery of the measurement target S is flaccid.

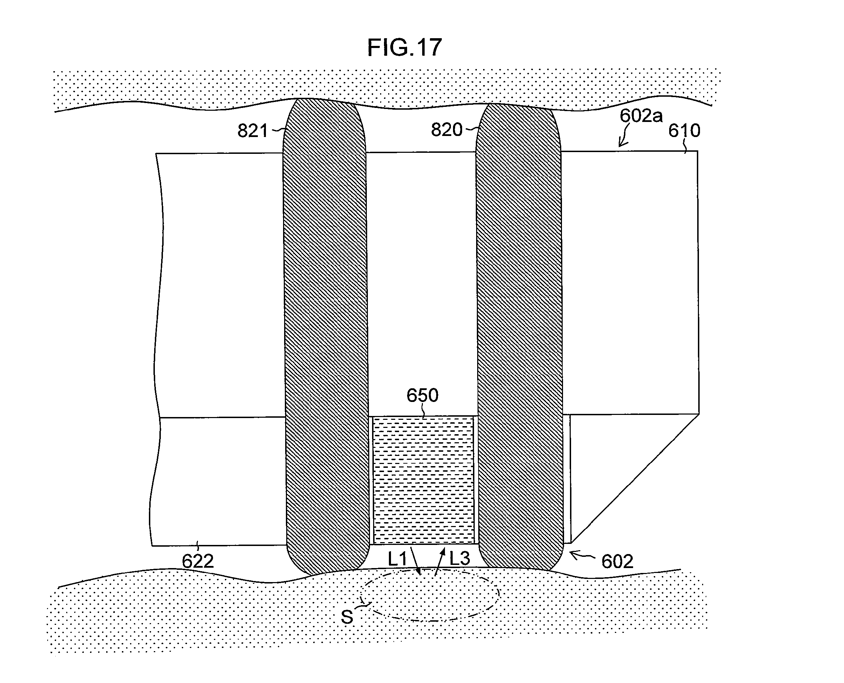

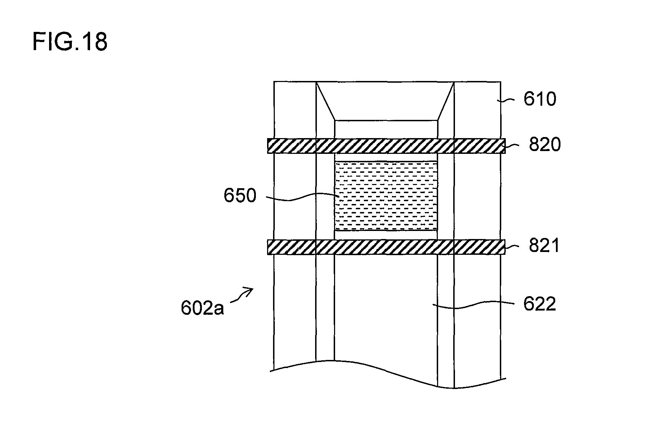

Figure 17 is a view that illustrates a third modification example of the distal end configuration of the OCT probe shown in Figure 12. Figure 18 is a view that illustrates the positional relationship between protrusions and an optical opening shown in Figure 17. As a third modification example of the present embodiment, as shown in Figure 17, instead of the balloons 700, 810, and 811, two ring-shaped balloons 820 and 821 are provided at positions at the front and rear of the optical opening 650 (see Figure 18). According to this configuration, when the balloon control portion 701 is operated to inflate the balloons 820 and 821 and fix the double sheath 602a, a similar action and effect as in the second modification example (see Figure 16) can be obtained.

### Fourth Embodiment:

The fourth embodiment is almost the same as the first embodiment. Hence only differences in the configuration thereof relative to the first embodiment are described hereafter, and like components are designated by like reference symbols and a description of such components is omitted.

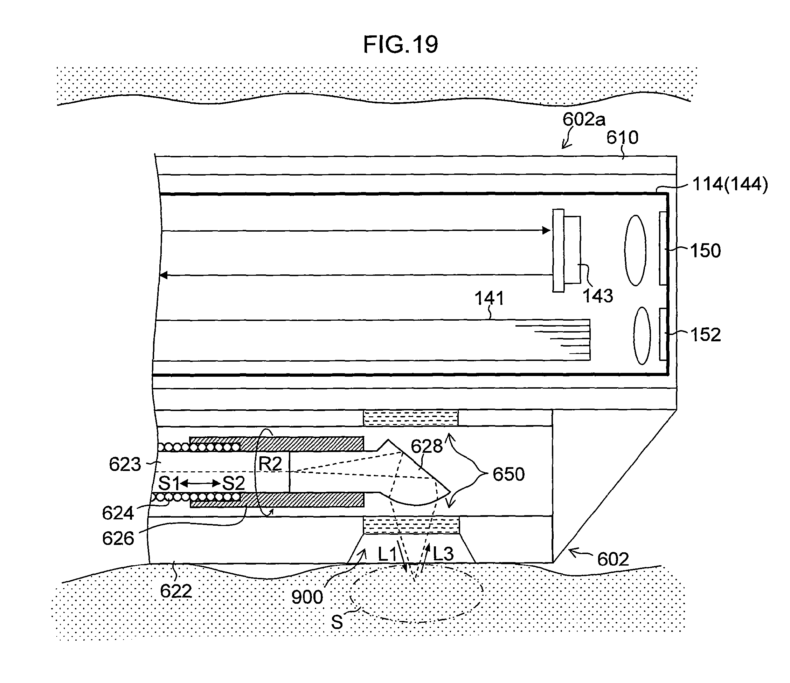

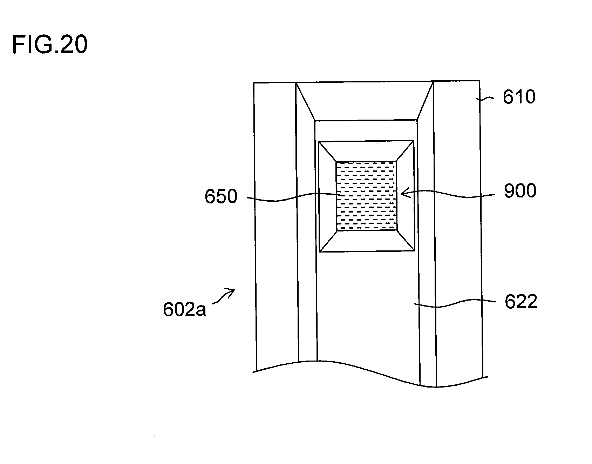

Figure 19 is a view that illustrates a distal end configuration of an OCT probe according to the fourth embodiment. Figure 20 is a view that illustrates a positional relationship between a concavity and an optical opening that are shown in Figure 19.

According to the present embodiment, as shown in Figure 19, a concavity 900 as a gap regulating means is formed by notching the circumference of the optical opening 650 of the OCT sheath portion 622. The remaining configuration is the same as in the first embodiment.

As shown in Figure 19, similarly to the first embodiment, according to the present embodiment the physician first inserts the insertion portion 114 of the endoscope 100 into the tube member 610 of the double sheath 602a. Subsequently, while viewing an image obtained with the endoscope 100, the physician inserts the double sheath 602a, through which the insertion portion 114 of the endoscope 100 has been inserted, into the body of the patient. When the physician finds an affected area as the measurement target S based on the endoscopic image, the physician rotates the double sheath 602a to perform an adjustment to make the OCT sheath portion 622 side face in the direction of the measurement target S, and presses the optical opening 650 of the OCT sheath portion 622 towards the measurement target S. Because of the existence of the concavity 900, the optical opening 650 enters a state in which the optical opening 650 is poised over the measurement target S, with a distance of, for example, 1 mm therebetween. In this state, the physician moves the optical fiber 623 and the optical lens 628 that are inside the OCT sheath portion 622 in the longitudinal axis direction while rotating the optical fiber 623 and the optical lens 628, to thereby perform two-dimensional scanning. Since information regarding the depth direction is obtained by the OCT measurement, the result of the measurement process is that data regarding the three-dimensional structure of the measurement target S is obtained.

Thus, according to the present embodiment, in addition to the advantageous effects of the first embodiment, an advantageous effect is obtained such that a distance between the optical opening 650 and the measurement target S can be regulated to a predetermined distance by the concavity 900 as a gap regulating means, to thereby ensure a gap therebetween. Consequently, in contrast to the first embodiment in which data regarding the three-dimensional structure of a deep layer portion of the measurement target S is acquired, according to the present embodiment, similarly to the third embodiment, for example, data regarding the three-dimensional structure of a surface layer portion of the measurement target S can be acquired. More specifically, according to the present embodiment, data regarding the three-dimensional structure of a layer portion of a desired depth of the measurement target S can be acquired based on the depth of the concavity 900 as a gap regulating means.

Note that although it is not illustrated in the drawings, the balloon 700 may also be provided according to the present embodiment, similarly to the second embodiment.

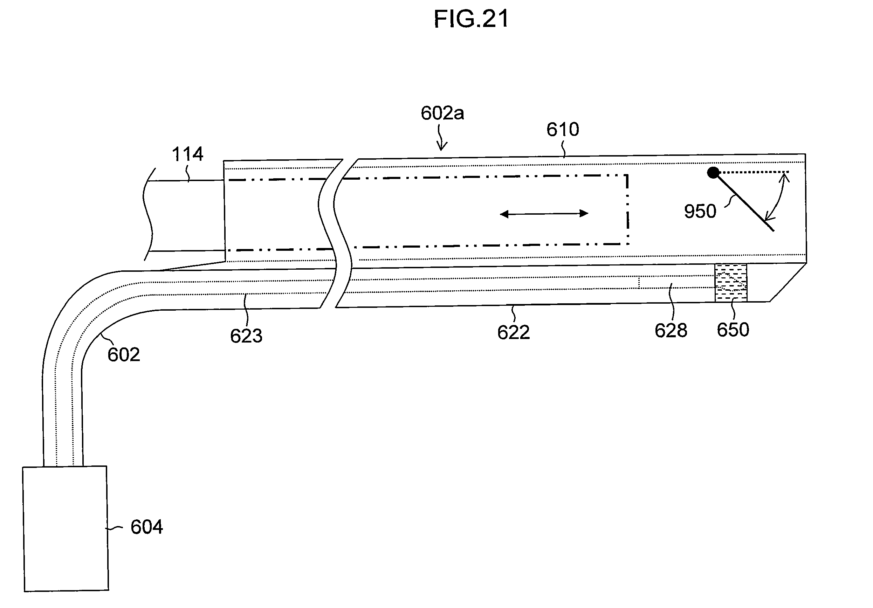

Figure 21 is a view that illustrates a configuration of a modification example of the tube member according to the first to fourth embodiments. As shown in Figure 21, it is more preferable to arrange an openable and closable mirror portion 950 at the distal end portion of the tube member 610 of each of the above embodiments. In this case, the mirror portion 950 is placed in an open state when inserting the tube member 610 into the body of a patient or when performing normal endoscope observation, so that the mirror portion 950 does not obstruct observation in the forward direction. The mirror portion 950 is placed in a closed state when pressing the optical opening 650 of the OCT sheath portion 622 against the measurement target S prior to OCT measurement. When the mirror portion 950 is in the closed state, the mirror portion 950 hangs down at a 45 degree angle at the front part of the endoscope. It is therefore possible to observe the bottom surface of the tube member 610 in which the optical opening 650 is provided, thus increasing the convenience of the physician when carrying out positioning or observation at a measurement location. Furthermore, an opening for endoscopic observation may also be formed in the tube member 610 at a position that is very close to the area in which the optical opening 650 is provided.

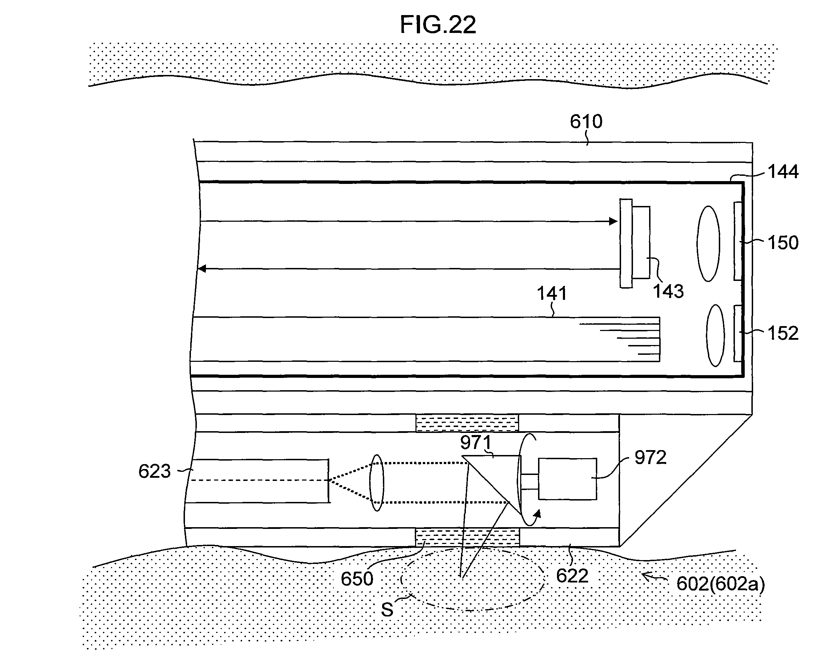

Note that although only radial scanning that rotates a fiber is described as an example of scanning according to each of the above embodiments, the present invention is not limited thereto, and configurations as shown in Figure 22 to Figure 25 may also be adopted.

Figure 22 is a configuration in which a deflection element 971 and a motor 972 that rotates the deflection element 971 are provided at a distal end of a probe. According to the scanning performed by this configuration, when the motor 972 rotates, a light beam is radially scanned by the deflection element 971.

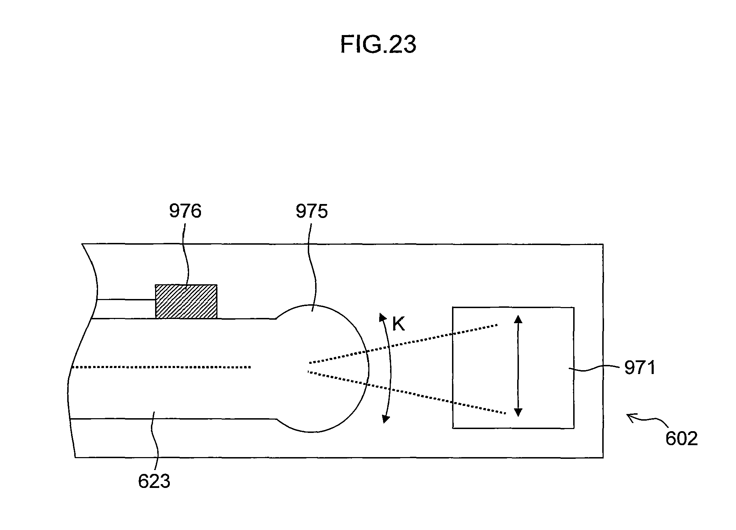

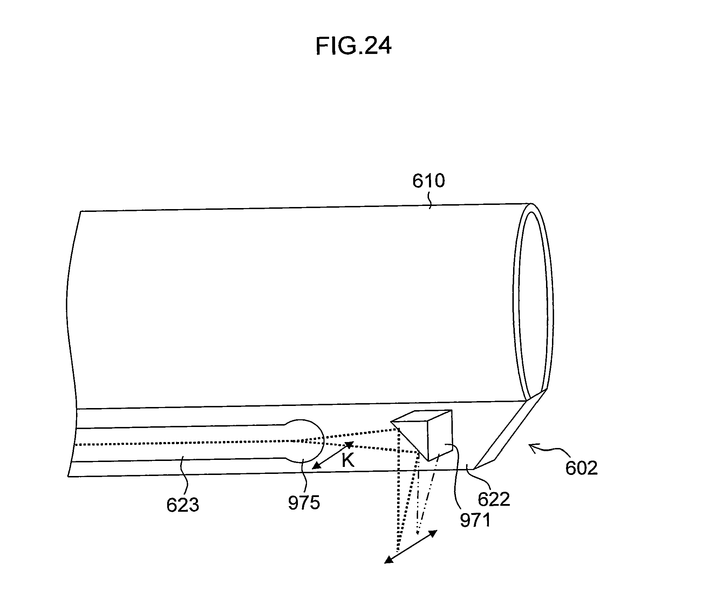

Figure 23 and Figure 24 illustrate a configuration in which a collimator lens 975 is integrally provided at a distal end of the optical fiber 623, and a piezo element (piezoelectric element) 976 is mounted at a position that is close to the distal end of the optical fiber 623. Figure 23 is a view that shows the optical fiber 623 as viewed from the tube member 610 side. Figure 24 is a side surface view of Figure 23. The scanning performed according to this configuration is a linear scan in which the optical fiber 623 is caused to swing in a left-to-right direction K by the piezo element 976. According to this configuration, when the piezo element 976 is caused to change shape by an electrical signal, in accompaniment therewith, the distal end of the optical fiber 623 vibrates in the left-to-right direction K. As a result, a light beam is scanned in a perpendicular direction to the optical axis of the optical fiber 623 via the deflection element 971.

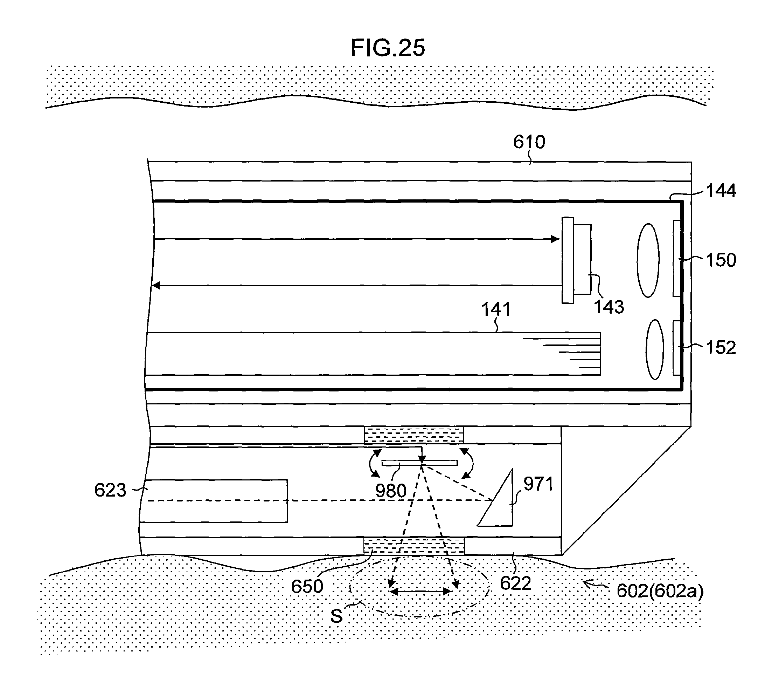

Figure 25 illustrates a linear-scan type configuration in which the OCT sheath portion contains a small micro-mirror 980. A light beam from the optical fiber 623 that travels via the deflection element 971 is linearly scanned by oscillating the micro-mirror 980. Although Figure 25 illustrates an example in which the micro-mirror 980 is oscillated in a direction that is parallel to the optical axis of the optical fiber 623, the oscillating direction is not limited thereto.

Note that although in each of the above described embodiments the optical opening 650 is constituted by a translucent material, a configuration may also be adopted in which the entire OCT probe 600 that includes the sheath 602a and the tube member 610 is constituted by a translucent material.

Although the OCT probe and the optical observation device of the present invention have been described in detail above, it should be understood that the present invention is not limited to the above examples. Naturally, various improvements and modifications may be made within a range that does not depart from the spirit and scope of the present invention.

### Reference Signs List

10... diagnostic imaging apparatus, 100... endoscope, 114, 602... insertion portion, 200... endoscope processor, 230... image synthesizing portion, 300... light source apparatus, 400... OCT processor, 500... monitor device, 600... OCT probe, 602a... double sheath, 610... tube member, 622... OCT sheath portion, 623... optical fiber, 628... optical lens, 650... optical opening, 700... balloon, 800... protrusion

## Claims

1. An optical probe, comprising:
a tubular sheath having a distal end that is closed;
a light guiding means provided inside the sheath, and arranged along a longitudinal axis of the sheath; and
a light condensing means that is provided on an emitting end side of the light guiding means, and that deflects an optical path of a light that is emitted from the emitting end so as to cause the light to converge on a measurement target;
wherein the optical probe scans a convergent light that is emitted from the light condensing means on a plane that is substantially orthogonal to an optical axis direction of the convergent light, and guides a light from the measurement target to the light guiding means via the light condensing means;
the optical probe further comprising a cylindrical tube member having an outer circumferential wall that is integrally joined to an outer wall of the sheath along a direction of the longitudinal axis.

2. The optical probe according to claim 1, wherein the tube member is an outer cylinder that has two ends that are open, and into which at least an endoscope insertion portion can be inserted.

3. The optical probe according to claim 1 or 2, wherein an inner surface shape of a cross section of the sheath that is orthogonal to the longitudinal axis of the sheath is a substantially trapezoidal shape.

4. The optical probe according to claim 1 or 2, wherein an inner surface shape of a cross section of the sheath that is orthogonal to the longitudinal axis of the sheath is a substantially semicircular shape.

5. The optical probe according to any one of claims 1 to 4, wherein at least the light condensing means is configured to be movable within a plane that is orthogonal to the longitudinal axis of the sheath inside the sheath.

6. The optical probe according to any one of claims 1 to 5, wherein the sheath has, at least at a distal end position of a side surface that faces an outer wall that is joined to the tube member, a translucent optical opening that transmits a light emitted from the light condensing means and a light from the measurement target.

7. The optical probe according to claim 6, further comprising:
a balloon means that is expandable and contractible that is provided at an outer circumferential position facing an outer circumferential side that is joined to the sheath of the tube member; and
a balloon control means that controls expansion and contraction of the balloon means.

8. The optical probe according to claim 6, wherein the sheath comprises a gap regulating means that is provided in a vicinity of the distal end position and that regulates a gap between an outer surface of the optical opening and a surface of the measurement target so that the gap is a predetermined distance.

9. The optical probe according to claim 8, wherein the gap regulating means is a protrusion that is provided at a side surface position that is adjacent to the optical opening.

10. The optical probe according to claim 9, wherein the protrusion is provided at front and rear of the optical opening along the longitudinal axis of the sheath.

11. The optical probe according to claim 7, comprising a gap regulating means that is provided in a vicinity of the distal end position and that regulates a gap between an outer surface of the optical opening and a surface of the measurement target so that the gap is a predetermined distance.

12. The optical probe according to claim 11, wherein the gap regulating means is a protrusion that is provided at a side surface position that is adjacent to the optical opening.

13. The optical probe according to claim 12, wherein the protrusion is provided at front and rear of the optical opening along the longitudinal axis of the sheath.

14. The optical probe according to claim 12 or 13, wherein the protrusion is a balloon member that is expandable and contractible.

15. The optical probe according to claim 14, wherein the balloon member is formed integrally with the balloon means.

16. The optical probe according to claim 14 or 15, wherein the balloon control means further controls expansion and contraction of the balloon member.

17. The optical probe according to claim 8 or 11, wherein:
the gap regulating means is a concavity that is formed at a position on a distal end side surface of the sheath; and
the optical opening is provided inside the concavity.

18. The optical probe according to any one of claims 1 to 17, wherein the closed distal end of the sheath is formed in a tapered shape.

19. An optical observation device, comprising:
an optical tomographic imaging apparatus that supplies a measuring light that is emitted to a measurement target inside a body cavity, and generates a tomographic image of the measurement target based on a light from the measurement target; and
an optical probe that guides the measuring light to the measurement target, and guides a light from the measurement target to the optical tomographic imaging apparatus;
the optical probe comprising:
a tubular sheath having a distal end that is closed;
a light guiding means that is provided inside the sheath, and that is arranged along a longitudinal axis of the sheath;
a light condensing means that is provided on an emitting end side of the light guiding means, and that deflects an optical path of a light emitted from the emitting end to cause the light to converge on a measurement target; and
a cylindrical tube member through which at least an elongated endoscope insertion portion having flexibility can be inserted, and which has an outer circumferential wall that is integrally joined to an outer wall of the sheath along a direction of the longitudinal axis;
wherein the optical probe scans a convergent light emitted from the light condensing means on a plane that is substantially orthogonal to an optical axis direction of the convergent light, and guides a light from the measurement target to the light guiding means via the light condensing means.

20. The optical observation device according to claim 19, further comprising:
a balloon means that is expandable and contractible that is provided at an outer circumferential position facing an outer circumferential wall that is joined to the sheath of the tube member; and
a balloon control portion that controls expansion and contraction of the balloon means.

21. The optical observation device according to claim 19 or 20, further comprising:
a translucent optical opening that is provided at least at a distal end position of a side surface that faces an outer wall that is joined to the tube member of the sheath, and that transmits a light emitted from the light condensing means and a light from the measurement target; and
a gap regulating means that is provided in a vicinity of the distal end position and that regulates a gap between an outer surface of the optical opening and a surface of the measurement target so that the gap is a predetermined distance.

22. An optical observation device, comprising:
an optical tomographic imaging apparatus that supplies a measuring light that is emitted to a measurement target inside a body cavity, and generates a tomographic image of the measurement target based on a light from the measurement target;
an optical probe that guides the measuring light to the measurement target, and guides a light from the measurement target to the optical tomographic imaging apparatus;
a light source means that supplies an illuminating light that is emitted to the measurement target;
an endoscope that comprises an elongated insertion portion that has flexibility, and that picks up an image of the measurement target to which the illuminating light is emitted; and
an endoscope processor that carries out signal processing with respect to an image pickup signal from the endoscope, and generates an endoscopic image of the measurement target;
the optical probe comprising:
a tubular sheath having a distal end that is closed;
a light guiding means that is provided inside the sheath, and that is arranged along a longitudinal axis of the sheath;
a light condensing means that is provided on an emitting end side of the light guiding means, and that deflects an optical path of a light emitted from the emitting end to cause the light to converge on a measurement target; and
a cylindrical tube member through which at least the insertion portion of the endoscope can be inserted, and which has an outer circumferential wall that is integrally joined to an outer wall of the sheath along a direction of the longitudinal axis;
wherein the optical probe scans a convergent light emitted from the light condensing means on a plane that is substantially orthogonal to an optical axis direction of the convergent light, and guides a light from the measurement target to the light guiding means via the light condensing means.

23. The optical observation device according to claim 22, further comprising:
a balloon means that is expandable and contractible that is provided at an outer circumferential position that faces an outer circumferential wall that is joined to the sheath of the tube member; and
a balloon control portion that controls expansion and contraction of the balloon means.

24. The optical observation device according to claim 22 or 23, further comprising:
a translucent optical opening that is provided at least at a distal end position of a side surface that faces an outer wall that is joined to the tube member of the sheath, and that transmits a light emitted from the light condensing means and a light from the measurement target; and
a gap regulating means that is provided in a vicinity of the distal end position and that regulates a gap between an outer surface of the optical opening and a surface of the measurement target so that the gap is a predetermined distance.
